(19) Europäisches Patentamt
European Patent Office
Office européen des brevets

(11) Publication number: **0 416 624 A2**

# (12) EUROPEAN PATENT APPLICATION

(21) Application number: **90117196.7**

(51) Int. Cl.[5]: **C08G 63/682**, C12P 7/42

(22) Date of filing: **06.09.90**

(30) Priority: **08.09.89 JP 233507/89**
**09.01.90 JP 1800/90**

(43) Date of publication of application:
**13.03.91 Bulletin 91/11**

(84) Designated Contracting States:
**AT BE DE DK FR GB IT NL**

(71) Applicant: **SHOWA DENKO KABUSHIKI KAISHA**
**13-9, Shiba Daimon 1-chome**
**Minato-ku, Tokyo 105(JP)**

(72) Inventor: **Doi, Yoshiharu**
**2617-39, Imajyuku-cho, Asahi-ku**
**Yokohama-shi, Kanagawa(JP)**
Inventor: **Fusho, Yuichi, c/o Showa Denko K.K.**
**Seikagaku Kenkyusho, 2-24-25, Tamagawa, Ota-ku**
**Tokyo(JP)**

(74) Representative: **Strehl, Schübel-Hopf, Groening**
**Maximilianstrasse 54 Postfach 22 14 55**
**W-8000 München 22(DE)**

## (54) Biodegradable or biocompatible copolymer and production process thereof.

(57) A copolymer comprising, as recurring units, (A) 1 to 99 mole% of one or more 3-hydroxyalkanoate unit having the formula (I):

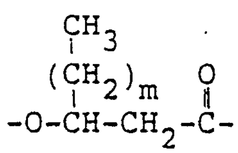

(I)

wherein m represents an integer of 0 or 1 to 8 and (B) 1 to 99 mole% of one or more 3-hydroxy-halogenated alkanoate unit having the following formula (II):

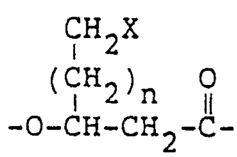

(II)

wherein X represents a halogen atom and n represents an integer of 2 to 8 and wherein the total of the unit (A) and the unit (B) is 100 mole%, and having a weight average molecular weight within the range of from 10,000 to 1,500,000.

EP 0 416 624 A2

Xerox Copy Centre

# BIODEGRADABLE OR BIOCOMPATIBLE COPOLYMER AND PRODUCTION PROCESS THEREOF

## BACKGROUND OF THE INVENTION

### 1. Field of the Invention

The present invention relates to a copolymer containing a 3-hydroxyalkanoate unit (hereinafter abbreviated as "3HA component" and a 3-hydroxy-halogenated alkanoate unit (hereinafter abbreviated as "3HAX component") and a process for the production thereof. More specifically, it relates to a novel copolymer containing halogen atoms produced by using a microorganism which can form and accumulate a poly-3-hydroxyalkanoate, and a process for the production thereof.

### 2. Description of the Related Art

Since Poly-3-hydroxybutyrate (PHB) is accumulated within cells of a large number of microorganisms, as an energy storing substance, and is a thermoplastic polymer exhibiting an excellent biodegradability or biocompatibility, it has attracted attention as a "clean" plastic. Particularly, under the present situation in which synthetic plastics are a serious social problem, from the standpoints of environmental pollution and resource circulation, PHB also has attracted attention as a biopolymer which does not depend on petroleum.

For example, PHB can be applied for medical materials such as operation threads or broken bone fixing materials, hygienic articles such as diapers or sanitary articles, agricultural or horticultural materials such as films for multiple purposes, slow release chemicals, fishery materials such as fishing nets, packaging materials and many other fields.

Nevertheless, PHB has a poor flexibility and can be worked only with difficulty, and this together with the problems of an inferior impact resistance and a high production cost, has meant that PHB has not been industrially produced on a practical scale.

Recently, studies have been made into a copolymer comprising a 3-hydroxybutyrate (hereinafter also referred to as "3HB") unit and a 3-hydroxyvalerate (hereinafter also referred to as "3HV") unit, and a process for the production thereof using Alcaligenes eutrophus (e.g., Japanese Unexamined Patent Publications (Kokai) Nos. 57-150393, 59-220192, and 63-269989).

Also, Japanese Unexamined Patent Publication (Kokai) No. 64-48821 discloses a copolymer comprising a 3-hydroxybutyrate unit and a 4-hydroxybutyrate (hereinafter also referred to as "4HB") unit, and a process for the production thereof. This publication discloses that copolymers exhibiting broad properties from a copolymer with a high crystallinity to a rubbery copolymer having a high elasticity can be obtained, depending on the content of 4-hydroxybutyrate.

Further, attempts have been made to produce new copolymers, particularly to introduce a functional group such as halogen atoms, hydroxyl groups, amino groups, etc., into polymers (Japanese Unexamined Patent Publications (Kokai) Nos. 57-150393, 58-69224), but an example of a copolymer having such functional groups as halogen atoms, etc., introduced therein has not been disclosed.

Also, an attempt to form 3HA by using hydrocarbon-utilizing the microorganism Pseudomonas oleovorans is described in Applied and Environmental Microbiology, 54, 1977 - 1982 (1988) or Macromolecules, 22, 1106 - 1115 (1989).

Further, Japanese Unexamined Patent Publication (Kokai) No. 63-226291 discloses a process for producing a polyester possessing a component having an unsaturated double bond together with a 3HA component in the monomer units, but does not disclose a polyester having a functional group such as halogen atoms.

As described above, among copolymers produced by microorganisms having biodegradability or biocompatibility characteristics, the prior art has not disclosed a copolymer, having halogen atoms as the functional group, which can be chemically modified or can be crosslinked with other polymer chains.

## SUMMARY OF THE INVENTION

Accordingly, the objects of the present invention are to eliminate the above-mentioned disadvantages of

the prior art and to provide a copolymer produced by a microorganism and having biodegradability or biocompatibility characteristics, and further, having a functional group which can be chemically modified or crosslinked with other polymer chains, and can be used as a starting material for a functional polymer having superior characteristics.

Other objects and advantages of the present invention will be apparent from the following description.

In accordance with the present invention, there is provided a copolymer comprising, as recurring units, (A) 1 to 99 mole% of at least one 3-hydroxyalkanoate unit having the formula (I):

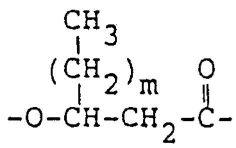

(I)

wherein m represents an integer of 0 or 1 to 8 and (B) 1 to 99 mole% of at least one 3-hydroxy-halogenated alkanoate unit having the formula (II):

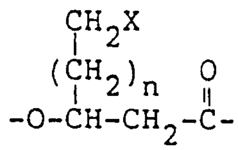

(II)

wherein X represents halogen (e.g., F, Cl, Br, I) and n represents an integer of 2 to 8, and wherein the total of the unit (A) and the unit (B) is 100 mole%, and having a weight average molecular weight of from 10,000 to 1,500,000.

In accordance with the present invention, there is also provided a process for producing the above-mentioned copolymer, which comprises culturing a microorganism capable of producing a poly-3-hydroxyalkanoate under at least one restricted condition of the addition amount of nitrogen and/or phosphorus and/or aeration amount in the presence of a compound having the following formula (III); or compounds having the formula (III) and (IV):

$CH_2X(CH_2)_pY$ (III)

$CH_3(CH_2)_rZ$ (IV)

wherein X represents a halogen atom; Y and Z each independently represent hydrogen or halogen (e.g., F, Cl, Br, I), hydroxyl or carboxyl or a metal salt thereof with 1 to 4-valent metal atom (e.g., Na, K, Mg, Ca, Al, Si) or a lower alkyl ester thereof (e.g., $C_1$-$C_4$ alkyl ester); p and r each independently represent an integer of 5 to 10, to thereby form and accumulate the poly-3-hydroxy-alkanoate within the microorganism cells, followed by recovering the thus-produced poly-3-hydroxyalkanoate.

## BRIEF DESCRIPTION OF THE DRAWINGS

The present invention will be better understood from the description set forth below with reference to the accompanying drawings, wherein;

Figure 1 shows the 125 MHz $^{13}$C-NMR spectrum of the copolymer obtained in Example 1;

Fig. 2 shows the 500 MHz $^{1}$H-NMR spectrum obtained in Example 1;

Fig. 3 and Fig. 4 are partially enlarged views of Fig. 1, wherein the numerals attached to the structural formula correspond to the numerals of the respective peaks;

Fig. 5 shows the 125 MHz $^{13}$C-NMR spectrum of the copolymer obtained in Example 6;

Fig. 6 shows the 500 MHz $^{1}$H-NMR spectrum of the copolymer obtained in Example 6; and

Fig. 7 shows the 250 MHz $^{19}$F-NMR spectrum of the copolymer obtained in Example 6, wherein the numerals of the peaks correspond to the numerals attached to the structural formula shown in Fig. 8.

## DESCRIPTION OF THE PREFERRED EMBODIMENTS

According to the present invention, the desired copolymer comprising a 3-hydroxyalkanoate unit having a halogen atom as the functional group and a 3-hydroxy-halogenated unit can be produced by cultivating a microorganism capable of producing a poly-3-hydroxyalkanoate in the presence of a halide compound, and accumulated within microorganism cells.

In the present invention, the 3HA component and the 3HAX component contained in the copolymer are represented by the following formulae, respectively:

3HA component: 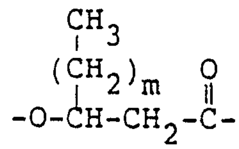

wherein m represents an integer of 0 or 1 to 8

3HAX component: 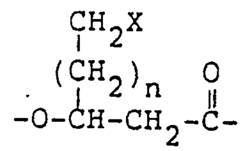

wherein X represents a halogen atom, and n an integer of 2 to 8

Specific examples of the 3HA component include:
a 3-hydroxybutyrate component (hereinafter referred to as "3HB component") when m = 0;
a 3-hydroxyhexanoate component (hereinafter referred to as "3HH component") when m = 2;
a 3-hydroxyoctanoate component (hereinafter referred to as "3HO component") when m = 4;
a 3-hydroxydecanoate component (hereinafter referred to as "3HD component") when m = 6;
a 3-hydroxydodecanoate component (hereinafter referred to as "3HDD component") when m = 8, and respectively represented by the following formulae:

3HB component: 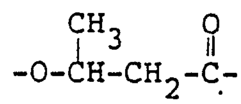

3HH component: 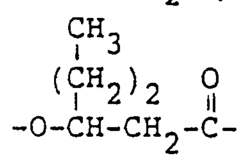

3HO component: 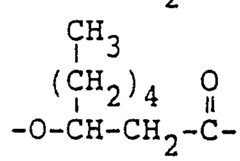

3HD component: 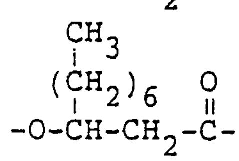

3HDD component: 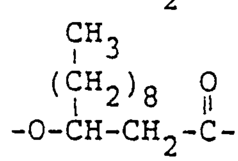

Specific examples of the 3HAX component include:
3-hydroxy-6-halogenated hexanoate (hereinafter also referred to as "3HH-6X component") when n = •2;
3-hydroxy-8-halogenated octanoate (hereinafter also referred to as "3HO-8X component") when n = 4;

3-hydroxy-10-halogenated decanoate (hereinafter also referred to as "3HD-10X component" when n = 6; 3-hydroxy-12-halogenated dodecanoate (hereinafter also referred to as "3HDD-12X component" when n = 8, and respectively represented by the following formulae:

3HH-6X component: 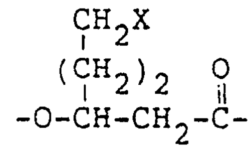

3HO-8X component: 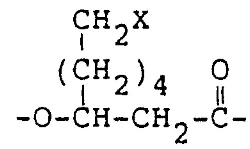

3HD-10X component: 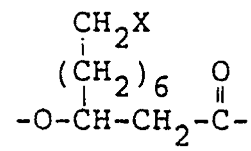

3HDD-12X component: 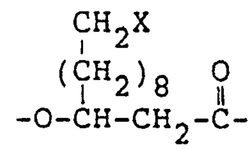

wherein X represents a halogen atom.

The copolymer comprising the 3HA component and the 3HAX component of the present invention, i.e., the copolymer comprising one or more respective 3HA components constituting the 3HA component and one or more respective 3HAX components constituting the 3HA component, is confirmed to be a random copolymer from the signal intensity ratios of the γ-position carbons of the respective chains of the monomer components constituting the copolymer in the $^{13}C$-NMR spectrum measurement.

The ratios of the 3HA component and the 3HAX component in the copolymer of the present invention, and the molecular weight of the copolymer, are variable depending on, for example, the microorganism employed for cultivation and the carbon sources and concentrations thereof in the culture medium, and cannot be defined unconditionally. Nevertheless, the former is variable with 1 to 99 mole%, preferably 20 to 95 mole%, of the 3HA component and 1 to 99 mole%, preferably 5 to 80 mole%, of the 3HAX component (with the proviso that the total of the 3HA component and the 3HAX component is 100 mole%), and weight average molecular weight of the latter is variable within the range of from about 10000 to 1,500,000, preferably 100,000 to 1,000,000.

The microorganism usable in the present invention is not particularly limited, provided that it is a microorganism capable producing a poly-3-hydroxyalkanoate, but in practice, for example, Pseudomonas oleovorans is employed. As the strain belonging to this species, for example, Pseudomonas oleovorans ATCC (American Type Culture Collection) 29347 and mutants thereof are particularly preferable.

The bacteriological properties of these microorganisms belonging to the genus Pseudomonas are described in, for example, "BERGEY's MANUAL OF DETERMINATIVE BACTERIOLOGY, Eighth Edition, The Williams & Wilkins Company/Baltimore". When culturing this microorganism, in the present invention, the compound of the formula (III) shown below, or the compounds of the following formulae (III) and (IV), are added:

$CH_2X(CH_2)_pY$ (III)

$CH_3(CH_2)_rZ$ (IV)

wherein X represents a halogen atom; Y and Z each independently represent a hydrogen atom, a halogen atom, a hydroxyl group, a carboxyl group or a metal salt thereof with a 1- to 4-valent atom, or a lower alkyl ester thereof; and p and r each independently represent an integer of 5 to 10.

This addition may be also made at the initial stage of cultivation, but preferably is made at the stage at which the growth has progressed to a certain extent.

In the following, the cultivation with an addition of the compound of the above-mentioned formula (III) or the compounds of the above-mentioned formula (III) and the above-mentioned formula (IV) from the initial stage of cultivation is described as the one-step cultivation, and the cultivation with an addition of the compound of the formula (III) or the compounds of the formula (III) and the formula (IV) from the stage when

growth has progressed to a certain extent is described as the two-step cultivation. In the latter case, the step at which growth has progressed to a certain extent is called the cultivation of the previous step, and the cultivation at the stage when the compound of the formula (III), or the compounds of the formula (III) and the formula (IV) is (are)added, to form and accumulate a copolymer within microorganism cells, is called the cultivation of the later step.

The cultivation is carried out under aerobic conditions, but in the one-step cultivation or in the cultivation of the later step, preferably the microorganism is cultured while restricting nitrogen and/or phosphorus, and/or amount of aeration. More specifically speaking, as is well-known in the art, when amounts of essential nutrient components (e.g., N, P, and/or $O_2$) in the culture medium or culture liquid are decreased, the proliferation of microorganisms is inhibited. Under such circumstances, when certain carbon sources are present, the polymer is accumulated in the medium as metabolites. Accordingly, it is preferable for the purpose of the present invention, i.e., for effectively producing and accumulating the polymer according to the present invention, that the addition amount of at least one essential nutrient component (i.e., N and/or P) and/or the aeration amount is restricted when the amount of the microorganism body in the culture medium or liquid is increased to a practically acceptable level (or concentration) (e.g., the logarithmic proliferation is effected until the $OD_{660}$ value becomes 20 to 30). Furthermore, specifically speaking, the amount of the nitrogen and/or phosphorus and/or the aeration amount are restricted, for example, such that the amount of nitrogen is 500 mg or less, in terms of an $NH_4$ ion, per 1 liter of the culture medium or liquid, the amount of phosphorus is 2.5 g or less, in terms of a $PO_4$ ion, per 1 liter of the culture medium or liquid, and the aeration amount is such that the dissolved oxygen content is 1 to 60% of the saturation amount for air. The desired result can be obtained only when at least one of the above-mentioned restricted conditions is fulfilled.

For the cultivation of the previous step, the conventional cultivation method of growing microorganisms is applicable, i.e., a medium and cultural conditions in which the microorganism to be used can be grown may be employed.

The culture medium components are not particularly limited, provided that they are substances which can be utilized by the microorganism to be used, but in practice, as the carbon sources there may be employed synthetic carbon sources such as octane, octanoic acid, methanol, ethanol and acetic acid, inorganic carbon sources such as carbon dioxide, natural products such as yeast extract, molasses, peptone and meat extract, saccharides such as arabinose, glucose, mannose, fructose and galactose, and sorbitol, mannitol and inositol. As nitrogen sources, for example, inorganic nitrogen compounds such as ammonia, ammonium salts, nitrates, and/or organic nitrogen containing compounds such as urea, corn steep liquor, casein, peptone, yeast extract, meat extract may be employed.

The inorganic components can be selected, for example, from calcium salts, magnesium salts, potassium salts, sodium salts, phosphoric acid salts, manganese salts, zinc salts, iron salts, copper salts, molybdenum salts, cobalt salts, nickel salts, chromium salts, boron compounds, and iodine compounds.

If necessary, vitamins can be employed.

The cultural conditions are not particularly limited, but the temperature is preferably, for example, about 20 to 40°C, more preferably about 25 to 35°C, and the pH is preferably, for example, about 5 to 10, more preferably about 6.5 to 9.5. The aerobic cultivation is carried out under these conditions.

When the cultivation is carried out in a range outside such conditions, the growth of the microorganism may be relatively poor, but the cultivation in a range outside such conditions will not be obstructed.

The cultivation system may be either batchwise or continuous.

In the cultivation of the later step, the cells obtained in the cultivation of the previous step are further cultured under the above-mentioned restricted conditions of the addition amount of nitrogen and/or phosphorus, and/or the the aeration amount.

More specifically, the microorganism cells are recovered by separation, by a conventional solid-liquid separation means such as filtration and centrifugation, from the culture broth obtained in the previous step, and the cells are subjected to cultivation in the later step, or alternatively, in the cultivation in the previous step, nitrogen and/or phosphorus is substantially depleted in the above-mantioned condition and/or aeration amount is restricted in the above-mentioned manner, and the culture broth can be migrated to cultivation in the later step without a recovery by separation of the cells to be cultured therein.

The cultivation in the later step is not different from the cultivation in the previous step, except that substantially no nitrogen and/or phosphorus is contained in the culture medium or the culture broth, and/or the aeration amount is restricted, and the compound represented by the above formula (III), or the compounds represented by the above formulae (III) and (IV) are contained as the carbon source.

Specific examples of the compound represented by the above formula (III) include halogenated aliphatic hydrocarbons such as 1-bromoheptane, 2-bromoheptane, 1-bromohexane, 1-bromooctane, 2-bromooctane;

1-chlorodecane; 1-chloroheptane, 1-chlorohexane, 1-chlorononane, 1-chlorooctane, 2-chlorooctane; 1-iododecane, 1-iodoheptane, 1-iodohexane, 1-iodononane, 1-iodooctane, 1-iodopentane, 1-iodoundecane; 1-fluorononane, 1-fluoropentane, 1-fluorooctane and the like; halogenated aliphatic alcohols such as 8-bromooctanol, 5-chloropentanol, 6-chloro-1-hexanol, 8-chloro-octanol, 10-chloro-1-decanol and the like; aliphatic carboxylic acids such as 8-bromooctanoic acid, 2-bromooctanoic acid, 8-chlorooctanoic acid and the like; and metal salts of carboxylic acids such as respective sodium, potassium, magnesium, calcium, aluminum salts of the above-mentioned carboxylic acids and the like; or esters of carboxylic acids such as methyl esters, ethyl esters of the above-mentioned carboxylic acids; and so on.

Specific examples of the compound represented by the above formula (IV) include aliphatic hydrocarbons such as hexane, heptane, octane, nonane, decane and the like; aliphatic alcohols such as hexanol, heptanol, octanol, nonanol, decanol and the like; aliphatic carboxylic acids such as hexanoic acid, heptanoic acid, octanoic acid, nonanoic acid, decanoic acid and the like; and metal salts of carboxylic acids such as the respective sodium, potassium, magnesium, calcium, aluminum salts of the above-mentioned carboxylic acids and the like; or esters of carboxylic acids such as the respective methyl esters, ethyl esters of the above-mentioned carboxylic acids; and so on.

The compound represented by the above formula (III) or the compounds represented by the above formulae (III) and (IV) are added and contained in the medium or the culture broth under cultivation.

The addition may be made at any point from the initial stage to the end stage, but initial stage is preferable in one-step cultivation and the initial stage of the cultivation of the later step is preferable in the two-step cultivation.

The amount of the compound of the above formula (III) or the compounds of the above formulae (III) and (IV) used may be an amount which can form the copolymer and will not inhibit the growth of the microorganism, and depends on the strain of the microorganism employed and the kinds of the monomer units or the ratios of the monomer units constituting the copolymer. Generally, the concentration of the compound of the above formula (III) or the compounds of the above formulae (III) and (IV) in the culture medium or culture broth are preferably about 0.1 to 20% w/v, more preferably about 1 to 5% w/v as the compound of the above formula (III) or the compounds of the above formulae (III) and (IV) per culture medium or culture broth.

In the one step cultivation or the cultivation in the later step, the carbon source can be made the only compound of the above formula (III) or the compounds of the above formulae (III) and (IV), but other carbon sources utilizable by the microorganism employed, such as glucose, fructose, methanol, ethanol, acetic acid, propionic acid, n-butyric acid, and lactose, also can be permitted to coexist in small amounts. For example, when glucose is permitted to coexist therein, the concentration of glucose is at most about 1.5 g/liter.

From the culture broth thus obtained, the microorganism cells are recovered by separation, by a conventional means such as filtration and centrifugation, and the cells are washed and dried to obtain dry cells. The copolymer formed is extracted from the dry cells or the wet cells in conventional manner, for example, by extraction with an organic solvent such as chloroform, and to the extract is added, for example, a poor solvent which will not substantially dissolve the copolymer such as methanol and ethanol, to thereby precipitate the copolymer.

Alternatively, the microorganism cells recovered by separation may be treated with a chemical which can solubilize the cells, such as sodium hypochlorite, or with an enzyme and/or a surfactant to solubilize the cell membrane and the cytoplasm of the cells, and the granular copolymer then recovered by separation by a conventional solid-liquid separation means, followed by washing and drying the copolymer.

## EXAMPLES

The present invention will now be further illustrated by, but is by no means limited to, the following Examples.

### Example 1

A copolymer was prepared by using of Pseudomonas oleovorans ATCC 29347.

#### Seed cultivation

Into a 500 ml Sakaguchi's flask were introduced 100 ml of a P medium (pH = 7.0) containing 2.5 g of n-octanoic acid per liter of medium, and the above mentioned Pseudomonas oleovorans was introduced and the culture was then shaken at 30° C for 48 hours.

The P medium contained the following components in one liter of deionized water.

| | |
|---|---|
| $(NH_4)_2HPO_4$ | 1.1 g |
| $K_2HPO_4$ | 5.8 g |
| $KH_2PO_4$ | 3.7 g |
| 100 mM $MgSO_4$ | 10 ml |
| Trace elements solution | 1 ml |

The above-mentioned trace elements solution contained the following components in one liter of 1 N-HCl.

| | |
|---|---|
| $FeSO_4 \cdot 7H_2O$ | 2.78 g |
| $MnCl_2 \cdot 4H_2O$ | 1.98 g |
| $CoSO_4 \cdot 7H_2O$ | 2.81 g |
| $CaCl_2 \cdot 2H_2O$ | 1.67 g |
| $CuCl_2 \cdot 2H_2O$ | 0.17 g |
| $ZnSO_4 \cdot 7H_2O$ | 0.29 g |

The main cultivation was carried out as described below.

Cultivation of previous step

Into a 5-liter jar fermentor was introduced 2-liter of the P medium (pH = 7.0) containing 2.5 g of n-octanoic acid per liter of the medium, and 20 ml of the culture broth obtained by seed cultivation was added thereto.

The cultivation was carried out at a pH pf 7.0 and an aeration amount of one liter/min. at 30° C for 24 hours, and during this period, 54 g/liter of sodium n-octanoate was successively added. The pH was adjusted with an aqueous sulfuric acid-ammonium sulfate solution.

Cultivation of later step

After completion of the cultivation of the previous step, 1-chlorooctane and n-octane were successively added, and the cultivation was carried out at a pH of 7.0 and an aeration amount of 1.0 liter/min. at 30° C for 24 hours.

The amounts of 1-chlorooctane and n-octane added were, respectively, 40 g/liter and 32.5 g/liter.

After cultivation, the microorganism cells were separated by centrifugation from the culture broth, to obtain the cells.

Treatment of microorganism cells

The cells obtained by the cultivation were washed with distilled water and then lyophilized to obtain dry cells.

Separation and recovery of copolymer

From the dry cells thus obtained, the copolymer was extracted with hot chloroform, precipitated by an addition of methanol to the extract, followed by a filtration and drying of the precipitates, to give the

copolymer.

Characteristics of copolymer

The composition, the molecular weight, the melting temperature, and the melting point heat of the copolymer thus obtained were measured as described below:

Composition: $^{1}H$-NMR spectrum and $^{13}C$-NMR spectrum

Molecular weight: GPC measurement

Melting temperature Tm: DSC measurement (temperature elevation speed: 10 $^{\circ}$C/min.)

Melting point ΔH: DSC measurement.

The determination results and the like are shown in Table 1.

The 125 MHz $^{13}C$-NMR spectrum of the copolymer obtained is shown in Fig. 1, and the 500 MHz $^{1}H$-NMR spectrum in Fig. 2.

For the copolymer obtained in this Example, the chain distribution of the copolymer was determined by using the 125 MHz $^{13}C$-spectrum.

Namely, according to the method of the present invention and others (Y. Doi et al. Macromolecules, 19, 2860-2864, (1986)), the dyad chain distributions of the respective components were determined from the multiplet resonance structure of the γ-position carbon.

Figure 3, Fig. 4 and Table 2 show the $^{13}C$-NMR measurement data, which show that the copolymer has a random copolymer chain distribution.

The calculated values of an elemental analysis of the copolymer obtained are C 57.3%, H 8.0% and Cl 15.0%, from the compositional ratios of the respective monomer units obtained from the $^{13}C$-NMR spectrum.

Example 2

Example 1 was repeated except that the aeration amount was changed to 0.3 liter/min. in the cultivation of the later step.

The measurement results are shown in Table 1.

The calculated values of the elemental analysis of the copolymer obtained are C 59.5%, H 8.4% and Cl 11.6%, from the compositional ratios of the respective monomer units obtained from the $^{13}C$-NMR spectrum.

Example 3

Example 1 was repeated except that the main cultivation was carried out as described below.

Namely, into a 2-liter Erlenmeyer's flask equipped with a baffle was introduced 500 ml of the P medium (pH = 7.0), 44 g/liter of 1-chlorooctane was added as the carbon source, and further, 1 ml of the culture broth obtained in the seed cultivation was added, followed by shaking the culture at 30 $^{\circ}$C for 48 hours.

The measurement results are shown in Table 1.

Example 4

Example 3 was repeated except that 22 g/liter-medium of 1-chlorooctane and 18 g/liter-medium of n-octane were used as the carbon sources in the main cultivation.

The measurement results are shown in Table 1.

Example 5

Example 3 was repeated except that 10 g/liter- medium of 1-chlorooctane and 27 g/liter-medium of n-octane were used as the carbon sources in the main cultivation.

The measurement results are shown in Table 1.

The calculated value of the elemental analysis of the copolymer obtained is Cl 6.0%, from the

compositional ratios of the respective monomer units obtained from the $^{13}C$-NMR spectrum.

Comparative Example 1

Example 3 was repeated except that 36 g/liter-medium of n-octane was used as the carbon source in the main cultivation, and the cultivation time was changed to 50 hours.

The measurement results are shown in Table 1.

The calculated values of elemental analysis of the copolymer obtained are C 67.3% and H 9.8%, from the compositional ratios of the respective monomer units obtained from the $^{13}C$-NMR spectrum.

Example 6

Example 1 was repeated except that the main cultivation was carried out as described below.

Cultivation of previous step

Into a 5-liter jar fermentor was introduced 2-liter of the P medium (pH = 7.0) containing 2.5 g of n-octanoic acid per liter of the medium, and 20 ml of the culture broth obtained by seed cultivation was added thereto.

The cultivation was carried out at a pH of 7.0 and an aeration amount of one liter/min. at 30 °C for 36 hours, and during this period, 59 g/liter of sodium n-octanoate was successively added. The pH was adjusted with an aqueous sulfuric acid, sodium hydroxide solution.

Cultivation of later step

After completion of the cultivation of the previous step, 1-fluorononane and n-nonane were successively added, and the cultivation was carried out at a pH of 7.0 and an aeration amount of 1.0 liter/min. at 30 °C for 28 hours.

The amounts of l-fluorononane and n-nonane were each 25 ml/liter.

The measurement results are shown in Table 3.

The 125 MHz $^{13}C$-NMR spectrum of the copolymer obtained is shown in Fig. 5, the 500 MHz $^{1}H$-NMR spectrum in Fig. 6, and the 250 MHz $^{19}F$-NMR spectrum in Fig. 7.

The calculated values of the elemental analysis of the copolymer obtained are C 67.9%, H 9.9%, and F 0.96%, from the compositional ratios of the respective monomer units obtained from the $^{13}C$-NMR spectrum.

Table 1

| Example or Comparative example | Carbon source g/liter medium | | Aeration amount | Weight of dry cells | Copolymer content in dry cells | Copolymer composition (mole %) | | | | | Molecular weight x 10^3 | | Mn / Mw | Melting temperature | Melting point | Elemental analysis (%) | | |
|---|---|---|---|---|---|---|---|---|---|---|---|---|---|---|---|---|---|---|
| | 1-Chloro-octane | n-Octane | ℓ/min | g/ℓ medium | (wt.%) | 3HO *1 | 3HO-8Cl *4 | 3HH *2 | 3HH-6Cl *5 | 3HB *3 | Mn *6 | Mw *7 | | (°C) | J\g | C | H | Cl |
| Example 1 | 40 | 32.5 | 1.0 | 2.6 | 5 | 29 | 59 | 2 | 10 | 0 | 87 | 201 | 2.3 | No remarkable peak | 0 | 57.1 | 7.9 | 16.7 |
| Example 2 | 40 | 32.5 | 0.3 | 2.3 | 10 | 45 | 40 | 4 | 11 | 0 | 80 | 210 | 2.6 | No remarkable peak | 0 | 59.6 | 8.4 | 12.8 |
| Example 3 | 44 | 0 | - | 0.2 | 2 | 29 | 50 | 0 | 0 | 21 | 254 | 874 | 3.4 | - | - | - | - | - |
| Example 4 | 22 | 18 | - | 0.7 | 1 | 27 | 69 | 0 | 0 | 4 | 75 | 254 | 3.4 | - | - | - | - | - |
| Example 5 | 10 | 27 | - | 0.5 | 2 | 26 | 20 | 0 | 0 | 54 | - | - | - | - | - | - | - | 7.4 |
| Comparative Example 1 | 0 | 36 | - | 1.4 | 19 | 91 | 0 | 9 | 0 | 0 | 90 | 177 | 2.0 | - | 13.6 | 66.9 | 9.8 | 0.2 |

*1) 3HO: 3-hydroxyoctanoate
*2) 3HH: 3-hydroxyhexanoate
*3) 3HB: 3-hydroxybutyrate
*4) 3HO-8Cl: 3-hydroxy-8-chlorooctanoate
*5) 3HH-6Cl: 3-hydroxy-6-chlorohexanoate
*6) Mn: number average molecular weight
*7) Mw: weight average molecular weight

Table 2

| Carbon Peak No. | Chemical shift ppm | Chain | Relative intensivity | |
|---|---|---|---|---|
| | | | Found *1 | Calcd. *2 |
| 26 (a) | 31.16 | 3HO • *3HH •6Cl + 3HH • *3HH-6Cl | 0.03 | 0.03 |
| (b) | 31.21 | 3HO-8Cl *3HH-6Cl + 3HH-6Cl • *3HH-6Cl | 0.07 | 0.07 |
| 12 (c) | 33.61 | 3HO • *3HO - 8Cl + 3HH • *3HO-8Cl | 0.16 | 0.18 |
| (d) | 33.69 | 3HO - 8Cl • *3HO-8Cl + 3HH-6Cl • *3HO-8Cl | 0.42 | 0.41 |
| 4 (e) | 33.77 | 3HO • *3HO + 3HH • *3HO | 0.12 | 0.09 |
| (f) | 33.86 | 3HO-8Cl • *3HO + 3HH-6Cl • *3HO | 0.18 | 0.20 |
| 20 | 35.90 | 3HO • *3HH + 3HH • *3HH<br>3HO-8Cl • *3HH + 3HH-6Cl • *3HH | 0.02 | 0.006<br>0.014 |

*1): determined from $^{13}$C-NMR
*2): chain distribution expected in the case of a random copolymer

Table 3

| No. | Carbon source ml/ℓ medium: 1-fluoro-nonane | Carbon source ml/ℓ medium: n-nonane | Aeration amount ℓ/min | Weight of dry cells g/ℓ | Copolymer content in dry cells (wt%) | Copolymer composition (mole %): 3HN *8 | 3HFN *9 | 3HO *1 | 3HE *10 | 3HFE *11 | 3HH *2 | Molecular weight x $10^3$: Mn *6 | Mw *7 | Mn/Mw | Melting temp. (°C) | Melting point J\g | Elemental analysis (%): C | H | F |
|---|---|---|---|---|---|---|---|---|---|---|---|---|---|---|---|---|---|---|---|
| Example 6 | 25 | 25 | 1.0 | 11.2 | 16.6 | 63 | 6.1 | 17.6 | 9.9 | 1.5 | 1.9 | 101 | 202 | 2.0 | 53.4 | 20.3 | 66.6 | 9.9 | 1.8 |

*1) 3HO: 3-hydroxyoctanoate
*2) 3HH: 3-hydroxyhexanoate
*6) Mn: number average molecular weight
*7) Mw: weight average molecular weight
*8) 3HN: 3-hydroxynonanoate
*9) 3HFN: 3-hydroxy-9-fluorononanoate
*10) 3HE: 3-hydroxyheptanoate
*11) 3HFE: 3-hydroxy-7-fluoroheptanoate

As mentioned above, according to the preparation process of the present invention, a useful functional group of halogen atom is introduced into the monomer unit of the copolymer, whereby a novel reactive copolymer can be obtained.

Further, the copolymer of the present invention can be applied not only for medical materials such as operation threads, broken bone fixing materials, slow release preparations, etc., hygienic materials such as diapers, sanitary articles, etc., agricultural and horticultural materials such as films for multiple purposes, slow release chemicals, etc., fishery materials such as fishing nets, etc., packaging materials and others, but also, because it has a halogen atom in the monomer unit in the copolymer, can be expected to introduce a new functional group such as a hydroxyl group through the chemical reaction or crosslinked with other polymer chains.

Therefore, the copolymer obtained by the present invention can be applied in many fields, e.g., as a starting material for creating a new functional polymer having various superior characteristics by utilizing the halogen atom contained in the monomer unit in the copolymer, while utilizing the characteristics such as biodegradability or biocompatibility possessed by the copolymer produced by a microorganism.

## Claims

1. A copolymer comprising, as recurring units, (A) 1 to 99 mole% of at least one 3-hydroxyalkanoate unit having the formula (I):

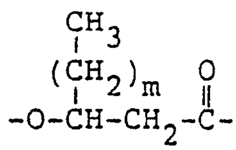

(I)

wherein m represents an integer of 0 or 1 to 8 and (B) 1 to 99 mole% of at least one 3-hydroxy-halogenated alkanoate unit having the formula (II):

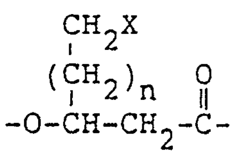

(II)

wherein X represents halogen and n represents an integer of 2 to 8 and wherein the total of the unit (A) and the unit (B) is 100 mole%
and having a weight average molecular weight within the range of from 10,000 to 1,500,000.

2. A copolymer as claimed in claim 1, wherein the copolymer comprises 20 to 95 mole% of the unit (A) and 5 to 80 mole% of the unit (B).

3. A copolymer as claimed in claim 1, wherein the weight average molecular weight of the copolymer is 100,000 to 1,000,000.

4. A process for producing a copolymer according to claim 1, which comprises culturing a microorganism capable of producing a poly-3-hydroxyalkanoate under at least one restricted condition of the addition amounts of nitrogen and phosphorus and the aeration amount in the presence of a compound having the following formula (III); or compounds having the formula (III) and (IV):

$CH_2X(CH_2)_pY$ (III)

$CH_3(CH_2)_rZ$ (IV)

wherein X represents a halogen atom; Y and Z each independently represent hydrogen, halogen, hydroxyl or carboxyl or a metal salt thereof with a 1 to 4-valent metal atom, or a lower alkyl ester thereof; and p and r each independently represent an integer of 5 to 10, to form and accumulate the poly-3-hydroxy-alkanoate within the microorganism cells, followed by recovering the poly-3-hydroxyalkanoate thus produced.

5. A process as claimed in claim 4, wherein said microorganism belongs to the genus Pseudomonas .

6. A process as claimed in claim 4, wherein said culturing is aerobically effected under the conditions of a

temperature of 20 to 40 °C and a pH of 6 to 10.

7. A process as claimed in claim 4, wherein the concentration of the compound (III) or a mixture of the compounds (III) and (IV) is 0.1 to 20% w/v in the culture medium.

8. A process as claimed in claim 4, wherein the addition amount of nitrogen or phosphorus is restricted, after the logarithmic proliferation is effected, such that an $OD_{660}$ of the culture medium or liquid is 20 to 30.

9. A process as claimed in claim 4, wherein the aeration amount is restricted, after the logarithmic proliferation is effected, such that the dissolved oxygen content is 1 to 60% of the saturation value for air.

Fig. 1

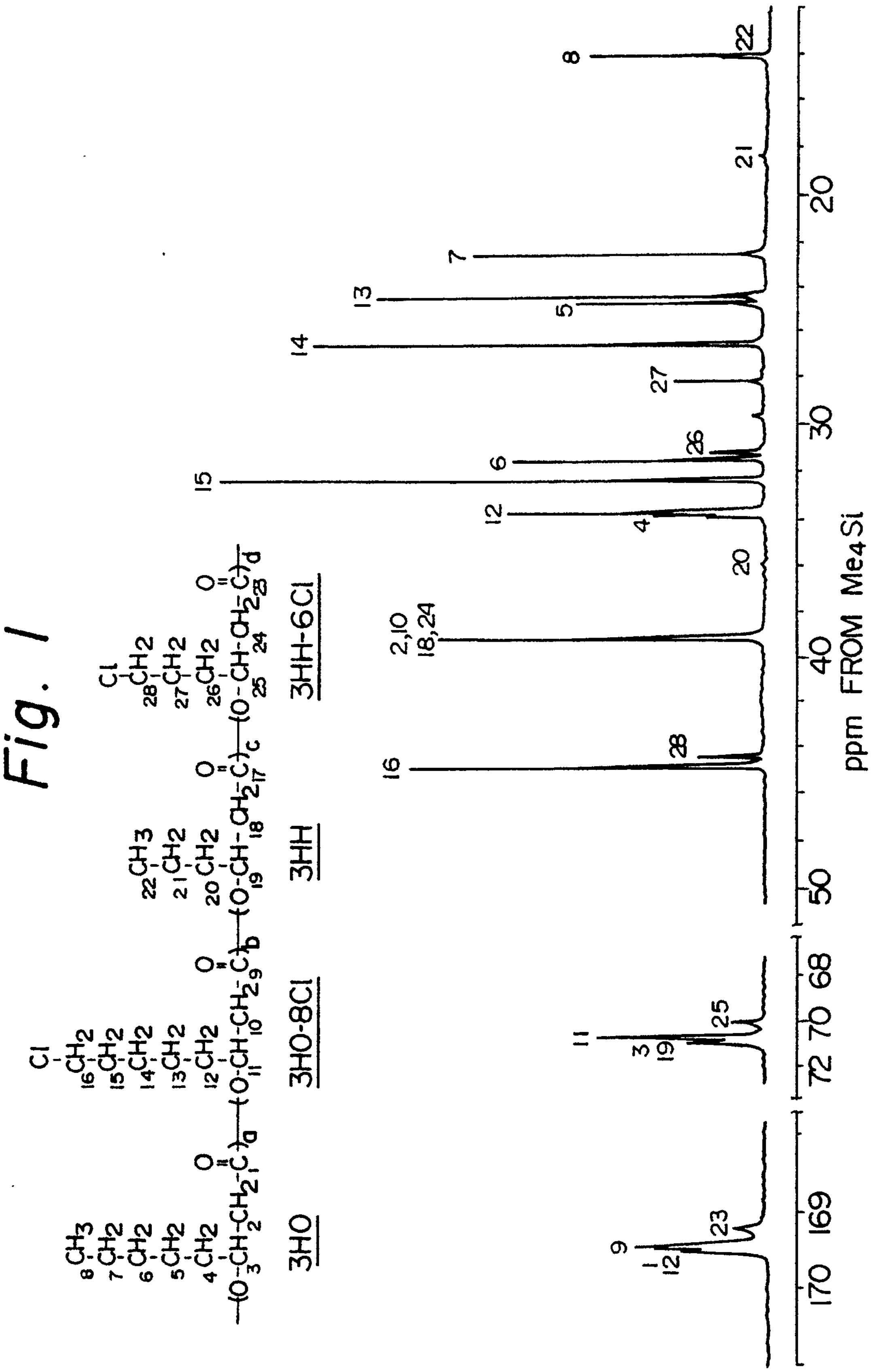
3HO
3HO-8Cl
3HH
3HH-6Cl
170 169
72 70 68
50 40 30 20
ppm FROM Me4 Si

Fig. 2

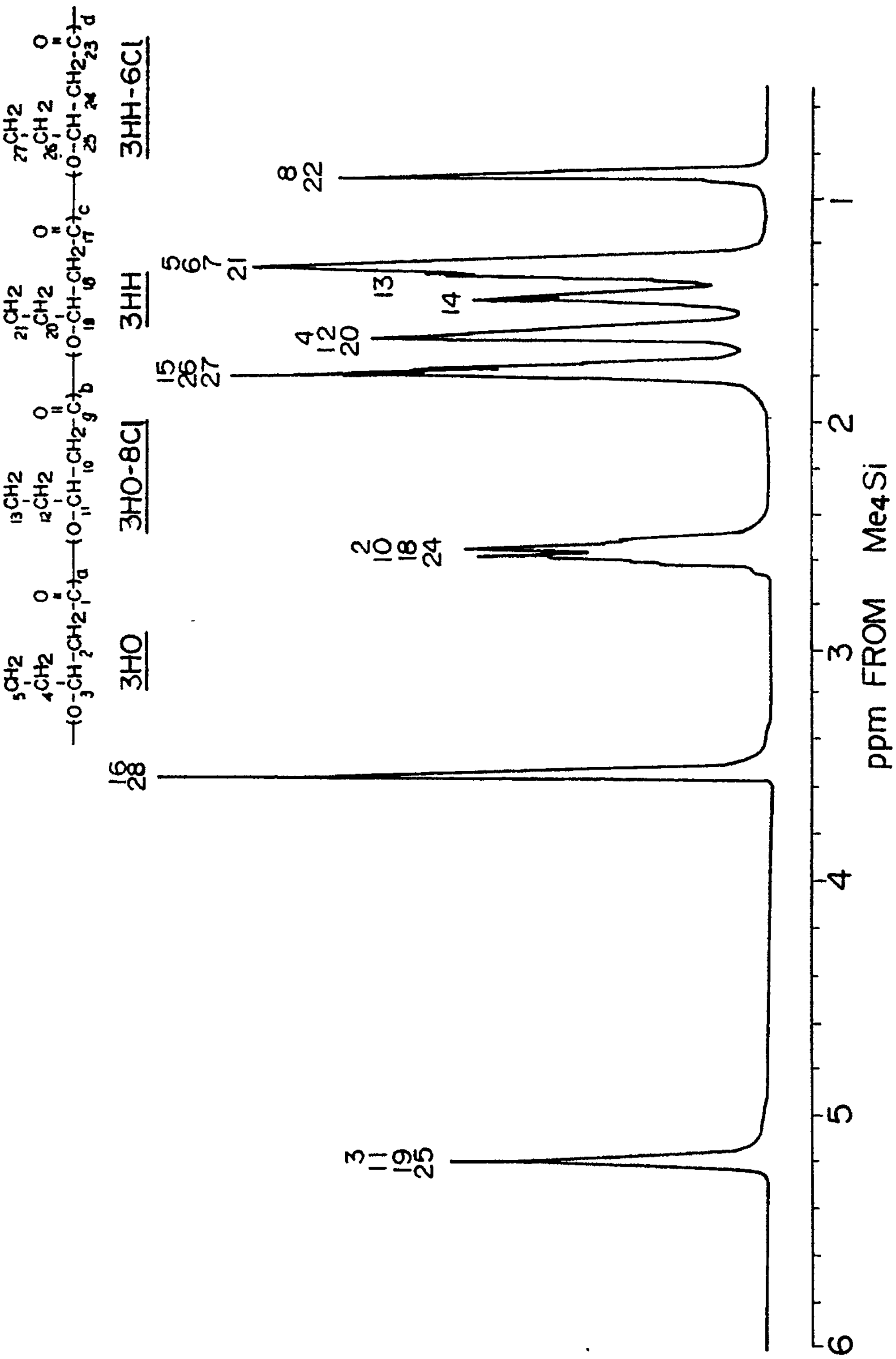

Fig. 3

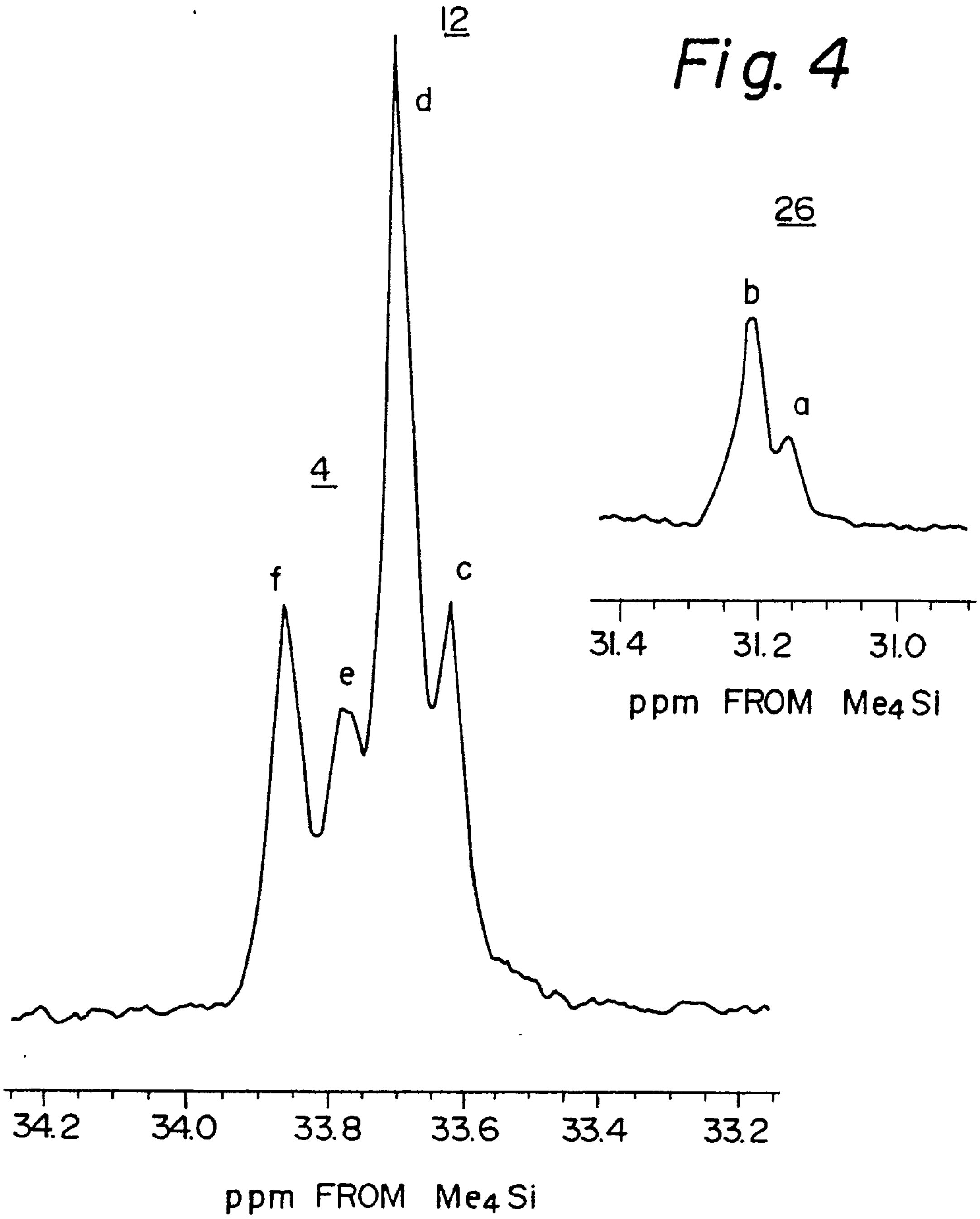
12
d
4
f
e
c
34.2
34.0
33.8
33.6
33.4
33.2
ppm FROM Me4 Si
Fig. 4
26
b
a
31.4
31.2
31.0
ppm FROM Me4 Si

# Fig. 5

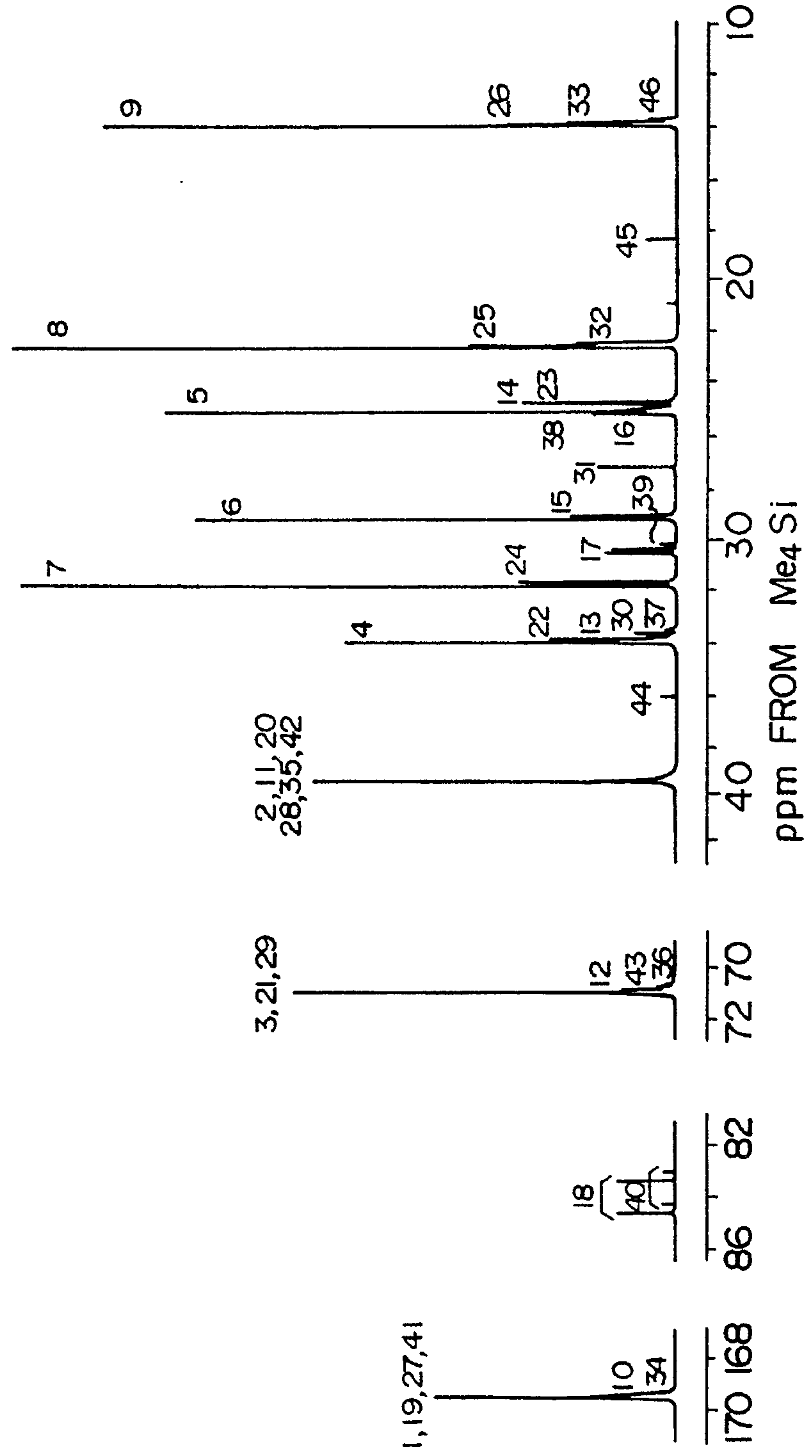
ppm FROM Me4 Si
10
20
30
40
72 70
82
86
170 168
9
26
33
46
45
8
25
32
5
14
23
38
16
31
6
15
39
7
24
17
4
22
13
30
37
44
2, 11, 20
28, 35, 42
3, 21, 29
12
43
36
18
40
1, 19, 27, 41
10
34

Fig. 6

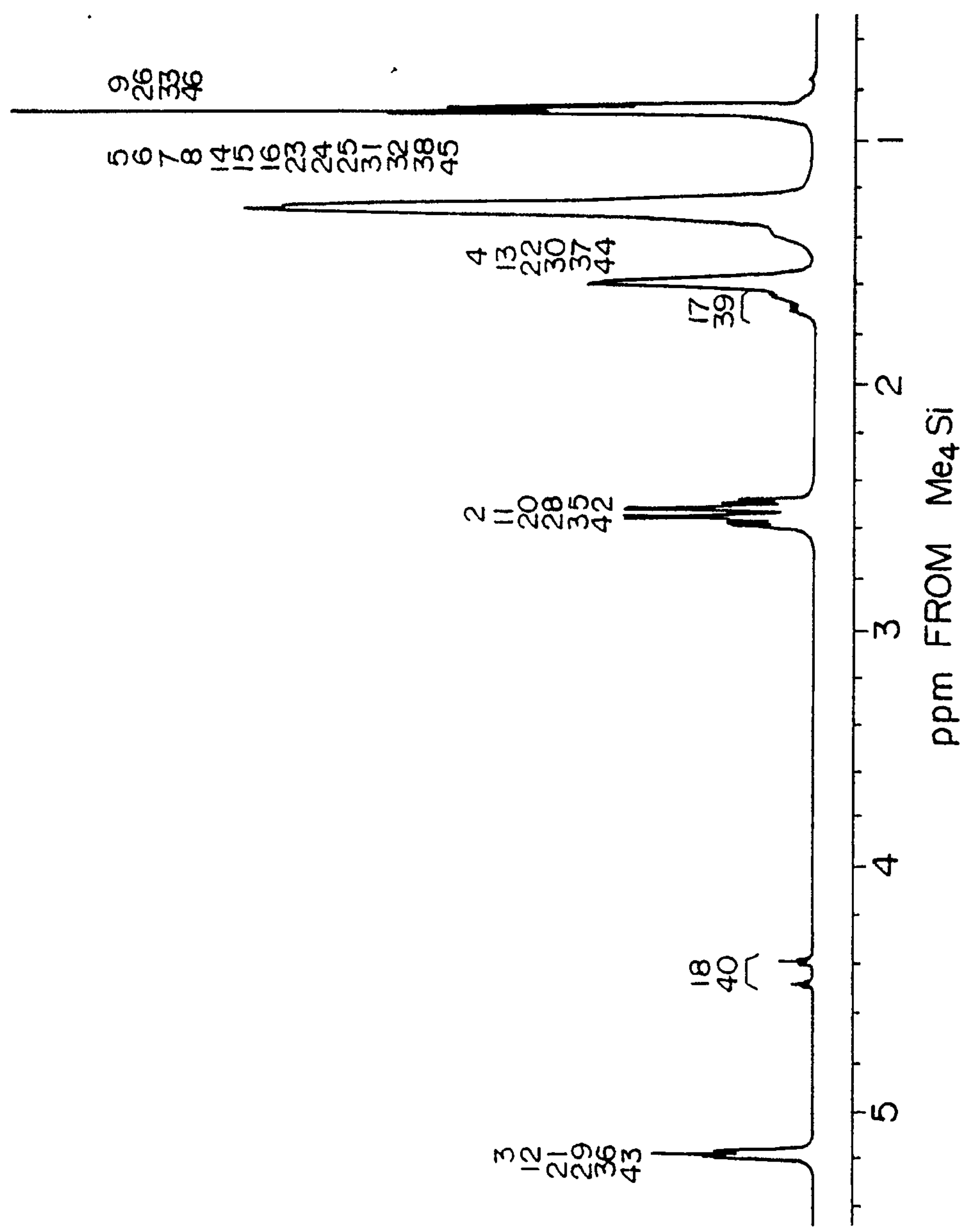

9 26 33 46
5 6 7 8 14 15 16 23 24 25 31 32 38 45
4 13 22 30 37 44
17 39
2 11 20 28 35 42
18 40
3 12 21 29 36 43
1
2
3
4
5
ppm FROM $Me_4$ Si

Fig. 7

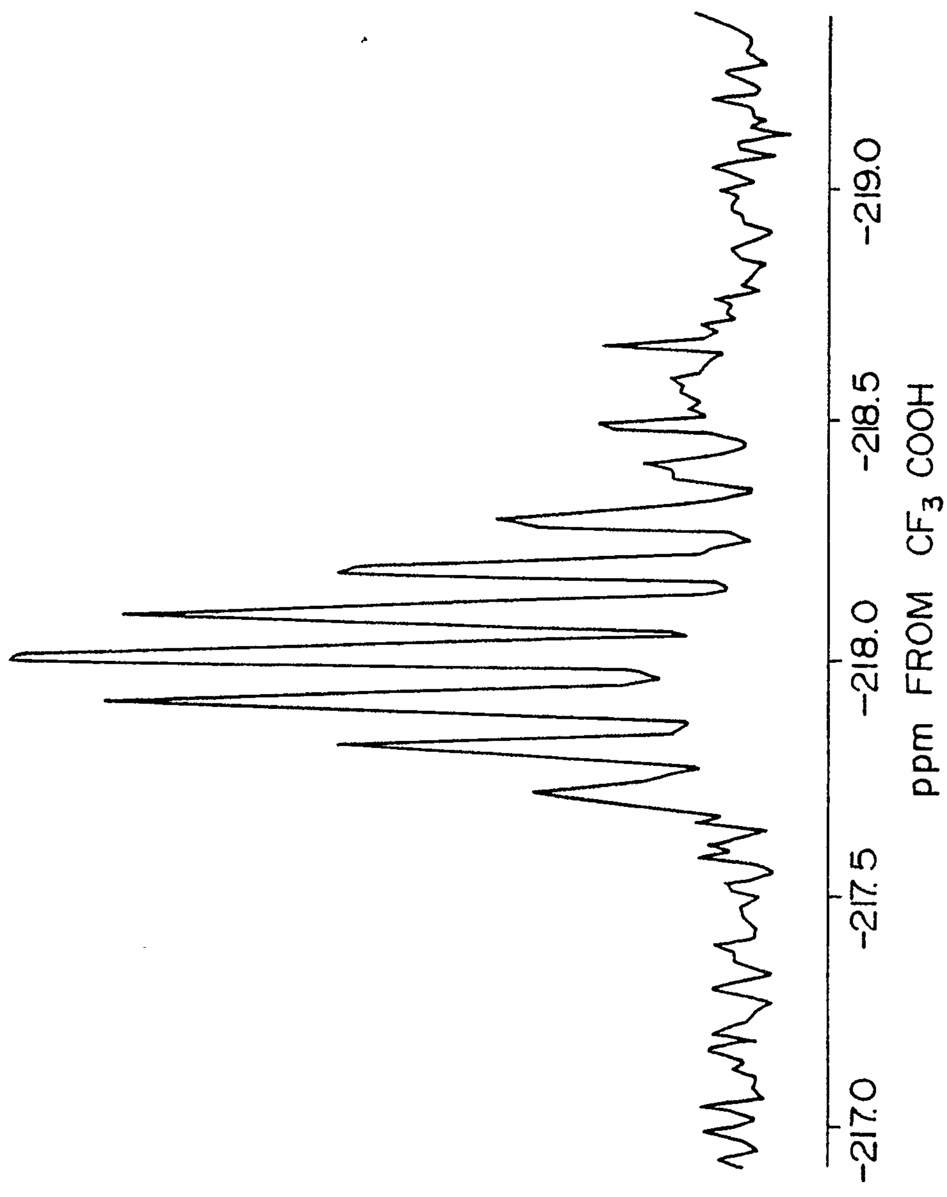

Fig. 8

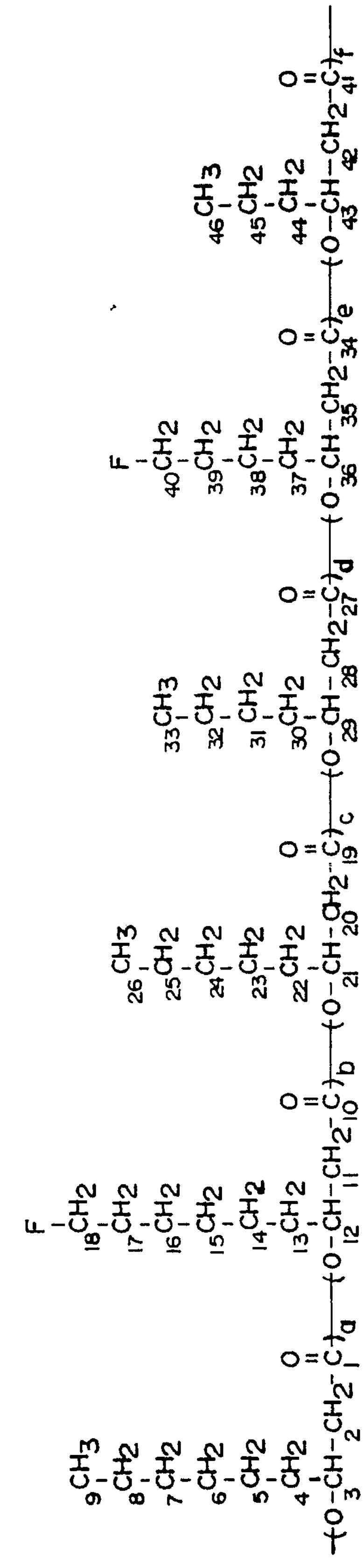

3HN 3HFN 3HO 3HE 3HFE 3HH